(19) Europäisches Patentamt
European Patent Office
Office européen des brevets

(11) EP 0 586 806 B1

(12) EUROPÄISCHE PATENTSCHRIFT

(45) Veröffentlichungstag und Bekanntmachung des Hinweises auf die Patenterteilung:
**06.10.1999 Patentblatt 1999/40**

(51) Int. Cl.$^6$: **A61K 31/195**, A61K 31/38, A61K 31/34, A61K 31/44, A61K 31/40, A61K 31/415, A61K 31/425

(21) Anmeldenummer: **93110222.2**

(22) Anmeldetag: **26.06.1993**

(54) **Verwendung von 2-(N-(2-Aminoethyl)amino)-essigsäure-derivaten zur Behandlung von Erkrankungen, die durch die nichtenzymatische Glykosylierung bedingt sind**

Use of 2-(N-(2-aminoethyl)amino)acetic acid derivatives for the treatment of diseases which are mediated by non-enzymatical glycosylation

Utilisation de dérivés du 2-(N-(2-Aminoethyl)amino)acide acétique dans le traitement des maladies liées à la glycosylation non-enzymatique

(84) Benannte Vertragsstaaten:
**AT BE CH DE DK ES FR GB GR IE IT LI NL PT SE**

(30) Priorität: **13.07.1992 DE 4222980**

(43) Veröffentlichungstag der Anmeldung:
**16.03.1994 Patentblatt 1994/11**

(73) Patentinhaber:
**HOECHST AKTIENGESELLSCHAFT**
**65926 Frankfurt am Main (DE)**

(72) Erfinder:
- **Schönafinger, Karl, Dr.**
  **D-8755 Alzenau (DE)**
- **Schindler, Ursula, Dr.**
  **D-6232 Bad Soden (DE)**
- **Schraven, Eckhard, Dr.**
  **D-6000 Frankfurt am Main 60 (DE)**

(56) Entgegenhaltungen:
**EP-A- 0 222 313** **EP-A- 0 316 852**
**WO-A-92/02216** **DE-A- 3 329 028**

- **ARZNEIM.-FORSCH. Bd. I, Nr. 1 , 1985 Seiten 93 - 102 R. BEYERLE ET AL. 'NEUE KARDIOVASCULÄR WIRKSAME 2-ARYL-2-IMIDAZOLINYL-ESSIGSÄUREN'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 586 806 B1

## Beschreibung

Die Erfindung betrifft die Verwendung von 2-(N-(2-Aminoethyl)amino)-essigsäure-derivaten der Formel I

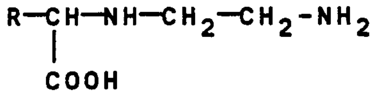

I

worin
R Phenyl, Thienyl, Furyl, Pyridyl, Pyrrolyl, Imidazolyl oder Thiazolyl, durch Halogen, Alkyl, Alkoxy, Dialkylaminoalkoxy, Alkoxyalkoxy ein oder mehrfach substituiertes Phenyl, Thienyl, Furyl, Pyridyl, Pyrrolyl, Imidazolyl oder Thiazolyl bedeutet und ihrer pharmakologisch annehmbaren Säureadditionssalze als pharmakologische Wirkstoffe, insbesondere zur Bekämpfung und Vorbeugung von Schäden oder Erkrankungen, die durch die nichtenzymatische Glykosylierung bedingt sind.

Die Erfindung betrifft auch die Verwendung der Verbindungen der Formel I und ihrer pharmakologisch annehmbaren Säureadditionssalze als pharmakologische Wirkstoffe zur Herstellung pharmazeutischer Präparate.

Die Erfindung betrifft auch pharmazeutische Präparate, die eine oder mehrere Verbindungen der Formel I und/oder ein oder mehrere pharmakologisch annehmbare Säureadditionssalze davon als Wirkstoffe enthalten.

Die für R stehenden Phenyl-, Thienyl-, Furyl-, Pyridyl-, Pyrrolyl-, Imidazolyl- oder Thiazolylreste können auch ein- oder mehrfach durch Halogen, Alkyl, Alkoxy, Dialkylaminoalkoxy und/oder Alkoxyalkoxy substituiert sein. Dabei kann ein Phenylrest vorzugsweise ein-, zwei- oder dreifach substituiert sein. Die übrigen für R stehenden Reste sind im Falle einer Substitution vorzugsweise einfach, insbesondere durch Alkyl oder Alkoxy, substituiert. Bei einer mehrfachen Substitution können die Substituenten gleich oder verschieden sein.

Halogen steht insbesondere für Fluor, Chlor, Brom und Iod, von denen Fluor und Chlor bevorzugt sind. Alkyl- und Alkoxyreste können, auch in Verbindung mit anderen Substituenten, geradkettig oder verzweigt sein.

Als Alkyl ist ($C_1$-$C_4$)Alkyl bevorzugt. Als Alkoxy ist ($C_1$-$C_4$)Alkoxy bevorzugt. Als Dialkylaminoalkoxy ist Di($C_1$-$C_4$)alkylamino-($C_1$-$C_4$)alkoxy bevorzugt. Als Alkoxyalkoxy ist ($C_1$-$C_4$)Alkoxy-($C_1$-$C_4$)alkoxy bevorzugt.

Der für R stehende Thienylrest ist vorzugsweise ein 2-Thienylrest. Der für R stehende Pyridylrest ist vorzugsweise ein 3-Pyridylrest. Der für R stehende Furylrest ist vorzugsweise ein 2-Furylrest. Der für R stehende Pyrrolylrest ist vorzugsweise ein 2-Pyrrolylrest. Der für R stehende Imidazolylrest ist vorzugsweise ein 5-Imidazolylrest. Der für R stehende Thiazolylrest ist vorzugsweise ein 4-Thiazolylrest.

Bevorzugte Reste R in der Formel I sind: Phenyl, 4-Methoxyphenyl, 4-Fluorphenyl, 4-Chlorphenyl, 3,4-Dimethoxyphenyl, 3,4,5-Trimethoxyphenyl, 4-(Diethylamino-ethoxy)-phenyl, 4-(Methoxy-ethoxy)-phenyl, 3-Pyridyl, 2-Furyl, 1-Methyl-imidazol-5-yl, Thiazol-4-yl, 2-Methyl-thien-5-yl, 1-Methyl-pyrrol-2-yl. 2-Thienyl ist für R besonders bevorzugt.

Von den Verbindungen der Formel I werden im Rahmen der vorliegenden Erfindung folgende bevorzugt verwendet:

2-(N-(2-Aminoethyl)amino)-2-(4-(2-diethylaminoethoxy)phenyl)-essigsäure-dihydrochlorid,
2-(N-(2-Aminoethyl)amino)-2-(4-fluorphenyl)-essigsäure-dihydrochlorid,
2-(N-(2-Aminoethyl)amino)-2-(4-methoxyphenyl)-essigsäure-dihydrochlorid,
2-(N-(2-Aminoethyl)amino)-2-(3,4,5-trimethoxyphenyl)-essigsäure-dihydrochlorid,
2-(N-(2-Aminoethyl)amino)-2-(3-pyridyl)-essigsäure-trihydrochlorid,
2-(N-(2-Aminoethyl)amino)-2-(4-chlorphenyl)-essigsäure-dihydrochlorid,
2-(N-(2-Aminoethyl)amino)-2-(2-furyl)-essigsäure-dihydrochlorid,
2-(N-(2-Aminoethyl)amino)-2-(1-methyl-imidazol-5-yl)-essigsäure-trihydrochlorid,
2-(N-(2-Aminoethyl)amino)-2-(4-thiazolyl)-essigsäure-dihydrochlorid,
2-(N-(2-Aminoethyl)amino)-2-(2-methyl-thien-5-yl)-essigsäure-dihydrochlorid,
2-(N-(2-Aminoethyl)amino)-2-(1-methyl-pyrrol-2-yl)-essigsäure-dihydrochlorid,
2-(N-(2-Aminoethyl)amino)-2-(4-(2-methoxyethoxy)phenyl)-essigsäure-dihydrochlorid,
2-(N-(2-Aminoethyl)amino)-2-(3,4-dimethoxy-phenyl)-essigsäure-dihydrochlorid,
2-(N-(2-Aminoethyl)amino)-2-(phenyl)-essigsäure-dihydrochlorid.

Im Rahmen der vorliegenden Erfindung wird die Verbindung 2-(N-(2-Aminoethyl)amino)-2-(2-thienyl)-essigsäure-dihydrochlorid besonders bevorzugt verwendet.

Da die Verbindungen der Formel I im Molekül sowohl eine saure Carboxylgruppe als auch zwei basische Gruppierungen enthalten, stellen sie zwitterionische Verbindungen dar, die innere Salze bilden können. Die Verbindungen

der Formel I können jedoch auch mit anderen anorganischen und organischen Säuren Salze bilden.

Geeignete Säuren sind beispielsweise Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipin-Säure. Pharmakologisch annehmbare Säureadditonssalze werden bevorzugt. Die Säureadditionssalze können wie üblich durch Vereinigung der Komponenten, zweckmäßigerweise in einem geeigneten Lösungs- oder Verdünnungsmittel, hergestellt werden.

Einige der Verbindungen der Formel I sind bereits als Zwischenprodukte zur Herstellung von cardiovaskulär wirksamen 2-Imidazolinyl-essigsäure-derivaten bekannt (vergl. Arzneimittel-Forsch./Drug Res. 35 (I), 1 (1985), Tabelle 1 und DE-A1-33 29 028, Beispiele 1b, 2b, 4b, 9 bis 20).

Überraschenderweise wurde nun gefunden, daß die Verbindungen der Formel I und ihre pharmakologisch annehmbaren Säureadditonssalze selbst pharmakologisch wirksam sind. Sie sind in der Lage, die nichtenzymatische Glykosylierung zu verhindern oder zu vermindern, d.h., z.B. zurückzudrängen oder zumindest günstig zu beeinflussen.

Die nichtenzymatische Glykosylierung ist für eine Reihe biologischer Effekte verantwortlich, so z.B. für die Inaktivierung von Proteinen, die Hemmung der Bindung von regulatorischen Molekülen, die Abfangung löslicher Proteine durch glykosilierte extrazelluläre Proteine, die Verminderung des Proteinabbaues, die abnormale DNA-Funktion, die mögliche immunogene Wirkung und Gewebetoxizität und die Quervernetzung glykosilierter Proteine. Die Vielzahl der von der nichtenzymatischen Glykosylierung beeinflußten biologischen Effekte läßt die pathologische Relevanz der nichtenzymatischen Glykosylierung erkennen.

Pathologische Veränderungen, die durch die nichtenzymatische Glykosylierung verursacht werden können, sind z.B. Osteoarthritis, Schlaganfall, Bluthochdruck, periphere Gefäßerkrankungen und Gelenksteifigkeit.

Wegen der Quervernetzung glykosilierter Proteine ist die nichtenzymatische Glykosylierung aber auch z.B. für die Entstehung diabetischer Spätschäden und altersbedingter Veränderungen verantwortlich.

Die Quervernetzung langlebiger Proteine wie z.B. Collagen, Crystallin, Elastin und Myelin nimmt mit zunehmendem Alter bei Mensch und Tier zu. Am Beispiel der Quervernetzung von Collagen konnte gezeigt werden, daß beim Krankheitsbild des Diabetes mellitus die altersbedingte Zunahme der "cross-links" deutlich beschleunigt ist. Diese Beobachtungen führten zur Hypothese, daß die vermehrte Ausbildung von Quervernetzungen von Collagen und anderer extrazellulärer Matrix-Proteine u.a. verantwortlich ist für die Entstehung physikalischer Änderungen von Membranen während des Alterns und für die chronischen Komplikationen beim Diabetes. Diese altersbedingten Veränderungen von Membranen im Zentralnervensystem können die Ursache von Lern- und Gedächtnisstörungen sein, die zum Nachlassen der geistigen Aktivität führen. Lern- und Gedächtnisstörungen findet man vor allem bei der Alzheimer'schen Krankheit, aber auch bei der Multi-Infarkt Demenz und der "gutartigen Alters-Vergeßlichkeit". Zu den diabetischen Spätschäden rechnet man u.a. Neuropathie, Nephropathie, Retinopathie, Katarakt, Atherosklerose/Arteriosklerose, Koagulopathie, Osteoporose und verminderte Elastizität des Bindegewebes. Intensive Untersuchungen lassen annehmen, daß die zentralen pathologischen Befunde des Diabetes ausgelöst werden durch die durch Hyperglämie beschleunigte Entstehung der späten Endprodukte der nichtenzymatischen Glykosylierungsreaktion im Gewebe. Bei der nichtenzymatischen Glykosylierung erfolgt eine nichtenzymatische Addition von reduzierenden bzw. reaktionsfähigen Zuckern, hauptsächlich von Glukose, an die freien

Aminogruppen von Proteinen. Kurzlebige Proteine (wie z.B. Enzyme, Albumine und Apoproteine) mit einer Halbwertszeit von Tagen oder Wochen reagieren mit reduzierenden bzw. reaktiven Zuckern, wie Glukose, unter Ausbildung einer Schiff'schen Base, wobei die Geschwindigkeit der Bildung der Schiff'schen Base von der Glukose-Konzentration im Blut abhängig ist:

$$\text{Glukose} + NH_2\text{-Protein} \underset{K_{-1}}{\overset{K_1}{\rightleftharpoons}} \text{Schiff'sche Base}$$

Die instabilen Schiff'schen Basen lagern sich dann innerhalb weniger Stunden bis Wochen zu den stabileren, jedoch reversiblen Amadori-Produkten um. Es stellt sich schließlich ein Blut-Glukose-abhängiges Gleichgewicht zwischen Schiff'scher Base und Amadori-Produkt ein:

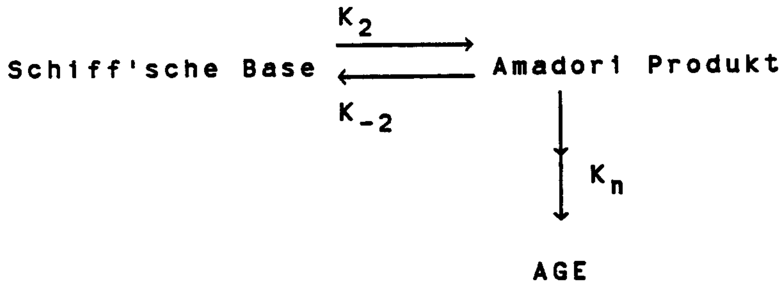


Einige der frühen Glykosilierungsprodukte des Collagens oder anderer langlebiger Proteine der Gefäßwand erfahren eine langsame komplexe Serie von Umwandlungsreaktionen, die schließlich zur Bildung der irreversiblen späten Glykosilierungs-Endprodukte führt. Diese irreversiblen späten Glykolisierungs-Endprodukte werden vorstehend und nachfolgend als AGEs ("**a**dvanced **g**lycosylation **e**ndproducts") bezeichnet. Diese AGEs verschwinden nicht mehr bei einer Korrektur des Blut-Glukose-Spiegels, sondern sie häufen sich während der gesamten Lebenszeit der Gefäßwand-Proteins kontinuierlich an und führen zu signifikanten strukturellen und funktionellen Veränderungen der Gefäßwand.

Die nichtenzymatische Glykosylierung ist jedoch nicht nur auf Proteine beschränkt. Auch Nukleinsäuren, wie z.B. DNA können in Gegenwart von Aminosäuren ebenfalls mit reduzierenden bzw. reaktiven Zuckern, insbesondere mit Glukose reagieren. Derartige Glukose-DNA-Produkte können für die vielfältigen altersbedingten Veränderungen des genetischen Materials verantwortlich sein, so z.B. für altersbedingte Schäden am zentralen Nervensystem (ZNS).

Die AGEs sind meist fluoreszierende, braune Pigmente, die sowohl in vitro wie auch in vivo nachgewiesen werden können. Wegen der braunen Farbe der AGEs wird die nichtenzymatische Glykosylierung z.B. auch als nichtenzymatische Bräunungsreaktion bezeichnet.

Einer medikamentösen Beeinflussung der nichtenzymatischen Glykosylierung kommt aus den vorgenannten Gründen eine hervorragende Rolle zu. Substanzen, die in der frühen Phase der nichtenzymatischen Glykosylierung eingreifen, könnten so auch die Bildung der AGEs beeinflussen. In vitro und in vivo Untersuchungen konnten einen derartigen Effekt für Aminoguanidin zeigen. Die Wirkungen der erfindungsgemäß zu verwendenden Verbindungen der Formel I sind bisher derjenigen von Aminoguanidin überlegen.

Die Verbindungen der Formel I und ihre pharmakologisch annehmbaren Säureadditionssalze sind daher in Form von Einzelverbindungen oder in Form von Mischungen untereinander als pharmakologische Wirkstoffe, insbesondere zur Bekämpfung, Linderung und Vorbeugung von Schäden oder Erkrankungen an Menschen und Tieren geeignet, die durch die nichtenzymatische Glykosylierung bedingt sind, so z.B. zur Bekämpfung oder Vorbeugung von Osteoarthritis, Schlaganfall, Bluthochdruck, peripheren Gefäßerkrankungen, Gelenksteifigkeit, von diabetischen Spätschäden (d.h., z.B. Neuropathie, Nephropathie, Retinopathie, Katarakt, Atherosklerose/Arteriosklerose, Koagulopathie Osteoporose und Verminderung der Elastizität des Bindegewebes), zur Bekämpfung oder Vorbeugung altersbedingter Veränderungen und altersbedingter Veränderungen des zentralen Nervensystems, wie z.B. von Lern- und Gedächtnisstörungen, altersbedingter Demenz und Alzheimer'sche Krankheit.

Die erfindungsgemäß zu verwendenden Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben einem oder mehreren üblichen pharmazeutisch einwandfreien Träger-, Füll- oder Verdünnungs- und gegebenenfalls einem oder mehreren Zusatzstoffen enthalten.

Die Heilmittel können oral, z.B. in Form von Tabletten, Filmtabletten, Dragees, Hart- und Weichgelatinekapseln, Mikrokapseln, Granulaten, Pulvern, Pellets, Lösungen, Sirupen, Emulsionen, Suspensionen, Aerosolen, Schäumen, Pillen oder Pastillen verabreicht werden. Die Verabreichung kann aber auch rektal, z.B. in Form von Suppositorien, oder parenteral, z.B. in Form von Injektionslösungen, oder perkutan, z.B. in Form von Salben, Cremes, Gelen, Pasten, Aerosolen, Schäumen, Pudern, Tinkturen, Linimenten oder sog. Transdermalen Therapeutischen Systemen (TTS) erfolgen.

Die pharmazeutischen Präparate können in an sich bekannter Weise unter Verwendung pharmazeutisch inerter anorganischer oder organischer Hilfs-, Träger-, Füll- oder Verdünnungsstoffe hergestellt werden. Für die Herstellung von Pillen, Tabletten, Filmtabletten, Dragees und die Pellet- oder Granulatfüllungen von Hartgelatinekapseln kann man z.B. Calciumphosphate, Lactose, Sorbitol, Mannitol, Stärken, präparierte Stärken, chemisch modifizierte Stärken, Stärkehydrolysate, Cellulose, Cellulosederivate, synthetische Polymere, Talk etc. verwenden. Träger- oder Verdünnungsstoffe für Weichgelatinekapseln und Suppositorien sind z.B. Fette, Wachse, halbfeste und flüssige Polyole, natürliche oder gehärtete Öle etc. Als Träger- oder Verdünnungsstoffe für die Herstellung von Lösungen und Sirupen eignen sich z.B. Wasser, Polyole, Lösungen von Saccharose, Invertzucker, Glukose etc. Als Trägerstoffe für die Herstellung von Injektionslösungen eignen sich z.B. Wasser, Alkohole, Glyzerol, Polyole oder pflanzliche Öle. Als Träger- oder Verdünnungsstoffe für Salben, Cremes und Pasten eignen sich z.B. Naturvaseline, Kunstvaseline, dick- und dünnflüssige Paraffine, Fette, natürliche oder gehärtete pflanzliche und tierische Öle, Neutralöle, Wachse, Wachsalkohole, Polyethylenglykole, Polyacrylsäure, Silicongele etc.

Die pharmazeutischen Präparate können in an sich bekannter Weise neben den Wirk- und Verdünnungs-, Füll- oder Trägerstoffen auch noch einen oder mehrere Zusatzstoffe oder Hilfsmittel, wie z.B. Spreng-, Binde-, Gleit-, Schmier-, Formtrenn-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler, Lösungsbeschleuniger, Antischaummittel, Salzbildner, Gelbildner, Verdickungsmittel, Fließregulierungsmittel, Sorptionsmittel, Mittel zur Erzielung eines Depoteffekts oder Mittel, insbesondere Salze, zur Veränderung des osmotischen Drucks, Überzugsmittel oder Antioxidantien etc. enthalten. Sie können auch zwei oder mehrere Verbindungen der Formel I oder ihrer pharmakologisch annehmbaren Säureadditionssalze und noch einen oder mehrere andere therapeutisch wirksame Stoffe enthalten.

Derartige andere therapeutisch wirksame Substanzen können beispielsweise sein: β-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

In den pharmazeutischen Präparaten kann der Gehalt an dem Wirkstoff oder den Wirkstoffen der Formel I in weiten Grenzen schwanken und z.B. 0,5 bis 90 Gew.%, vorzugsweise 1 bis 50 Gew.%, betragen. In festen Darreichungsformen, wie Dragees, Tabletten etc. beträgt der Gehalt an einem oder mehreren Wirkstoffen der Formel I in vielen Fällen 2 bis 80 Gew.%. Flüssige Darreichungsformen, wie Tropfen, Emulsionen und Injektionslösungen enthalten häufig 0,5 bis 20 Gew.%, vorzugsweise 0,5 bis 10 Gew.%, eines oder mehrerer Wirkstoffe der Formel I. Der Gehalt an einem oder mehreren Wirkstoffen der Formel I kann gegebenenfalls in den pharmazeutischen Präparaten teilweise, z.B. bis zu 50 Gew.%, vorzugsweise zu 5 bis 40 Gew.%, durch eine oder mehrere andere therapeutisch wirksame Substanzen ersetzt sein.

Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 1 bis 1000 mg, vorzugsweise 10 bis 400 mg, angemessen. Auch bei anderen Applikationsformen liegt die Tagesdosis in ähnlichen Mengenbereichen. Die Tagesdosis kann in mehrere, z.B. 2 bis 4 Teilverabreichungen aufgeteilt werden.

Soweit die Verbindungen der Formel I nicht bereits in der Literatur beschrieben sind, vergl. insbesondere DE-A1-33 29 028, Beispiele 1b, 2b, 4b, 9 bis 20, lassen sie sich nach den für diese Verbindungsklasse bekannten Herstellungsverfahren leicht synthetisieren. So können die Verbindungen der Formel I z.B. durch Eintragen von Verbindungen R-Cl, wobei R die bei der Formel I angegebene Bedeutung besitzt, in überschüssiges 1,2-Diaminoethan und anschließendes Erhitzen hergestellt werden, vergl. DE-A1-33 29 028, Beispiel 9. Sie können auch durch Hydrolyse von in 3-Stellung mit R substituierten Piperazin-2-onen hergestellt werden, vergl. DE-A1-33 29 028, Seite 14 und Beispiele 1b4, 2b2 und 4b, sowie Arzneim.-Forsch./Drug Res. 35 (I), 1 (1985).

Die nachfolgenden Beispiele betreffen pharmazeutische Zubereitungsformen

## Beispiel A

Gelatineweichkapseln, enthaltend 100 mg Wirkstoff pro Kapsel:

| | pro Kapsel |
|---|---|
| Wirkstoff | 100 mg |
| aus Kokosfett fraktioniertes Triclycerid-Gemisch | 400 mg |
| Kapselinhalt | 500 mg |

**Beispiel B**

Injektionslösung, enthaltend 20 mg Wirkstoff pro ml:

| | **pro ml** |
|---|---|
| Wirkstoff | 2,0 mg |
| Polyethylenglycol 400 | 5,0 mg |
| Natriumchlorid | 2,7 mg |
| Wasser zu Injektionszwecken | ad 1 ml |

**Beispiel C**

Emulsion, enthaltend 60 mg Wirkstoff pro 5 ml:

| | **pro 100 ml Emulsion** |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

**Beispiel D**

Rektale Arzneiform, enthaltend 40 mg Wirkstoff pro Suppositorium:

| | **pro Suppositorium** |
|---|---|
| Wirkstoff | 40 mg |
| Suppositoriengrundmasse | ad 2 g |

**Beispiel E**

Tabletten, enthaltend 40 mg Wirkstoff pro Tablette:

| | **pro Tablette** |
|---|---|
| Wirkstoff | 40 mg |
| Lactose | 600 mg |

(fortgesetzt)

| | pro Tablette |
|---|---|
| Maisstärke | 300 mg |
| lösliche Stärke | 20 mg |
| Magnesiumstearat | 40 mg |
| | 1000 mg |

**Beispiel F**

Dragees, enthaltend 50 mg Wirkstoff pro Dragee:

| | pro Dragee |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |
| | 260 mg |

**Beispiel G**

Für die Herstellung des Inhalts von Hartgelatinekapseln eignen sich die folgenden Rezepturen:

| | | |
|---|---|---|
| a) | Wirkstoff | 100 mg |
| | Maisstärke | 300 mg |
| | | 400 mg |
| b) | Wirkstoff | 140 mg |
| | Milchzucker | 180 mg |
| | Maisstärke | 180 mg |
| | | 500 mg |

**Beispiel H**

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |

(fortgesetzt)

| | |
|---|---|
| Benzoesäureethylester | 0,03 g |
| Ethanol 96 %ig | 5 ml |
| entmineralisiertes Wasser | ad 100 ml |

**Patentansprüche**

1. 2-(N-(2-Aminoethyl)amino)-essigsäurederivate der Formel I,

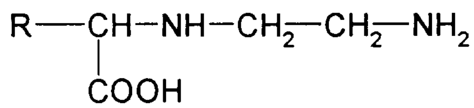

I

worin R Phenyl, Thienyl, Furyl, Pyridyl, Pyrrolyl, Imidazolyl, Thiazolyl oder durch Halogen, Alkyl, Alkoxy, Dialkylaminoalkoxy, Alkoxyalkoxy ein- oder mehrfach substituiertes Phenyl, Thienyl, Furyl, Pyridyl, Pyrrolyl, Imidazolyl oder Thiazolyl bedeutet, und ihre pharmakologisch annehmbaren Säureadditionssalze zur Verwendung als Arzneimittel.

2. 2-(N-(2-Aminoethyl)amino)-essigsäurederivate der Formel I gemäß Anspruch 1, dadurch gekennzeichnet, daß R Phenyl, Thienyl, Furyl, Pyridyl, Pyrrolyl, Imidazolyl, Thiazolyl oder durch Halogen, ($C_1$-$C_4$)-Alkyl, ($C_1$-$C_4$)-Alkoxy, Di-($C_1$-$C_4$)-alkylamino-($C_1$-$C_4$)-alkoxy, ($C_1$-$C_4$)-Alkoxy-($C_1$-$C_4$)-alkoxy ein- oder mehrfach substituiertes Phenyl, Thienyl, Furyl, Pyridyl, Pyrrolyl, Imidazolyl oder Thiazolyl bedeutet, und ihre pharmakologisch annehmbaren Säureadditionssalze zur Verwendung als Arzneimittel.

3. 2-(N-(2-Aminoethyl)amino)-essigsäurederivat der Formel I gemäß Anspruch 1 und/oder 2, dadurch gekennzeichnet, daß R 2-Thienyl bedeutet, und seine pharmakologisch annehmbaren Säureadditionssalze zur Verwendung als Arzneimittel.

4. Verwendung eines 2-(N-(2-Aminoethyl)amino)-essigsäurederivats der Formel I gemäß einem oder mehreren der Ansprüche 1 bis 3 oder eines pharmakologisch annehmbaren Säureadditionssalzes davon zur Herstellung eines Arzneimittels zur Bekämpfung oder Vorbeugung von Schäden oder Erkrankungen, die durch nichtenzymatische Glykolysierung hervorgerufen werden.

5. Verwendung eines 2-(N-(2-Aminoethyl)amino)-essigsäurederivats der Formel I gemäß einem oder mehreren der Ansprüche 1 bis 3 oder eines pharmakologisch annehmbaren Säureadditionssalzes davon zur Herstellung eines Arzneimittels zur Bekämpfung oder Vorbeugung von diabetischen Spätschäden.

6. Verwendung eines 2-(N-(2-Aminoethyl)amino)-essigsäurederivats der Formel I gemäß einem oder mehreren der Ansprüche 1 bis 3 oder eines pharmakologisch annehmbaren Säureadditionssalzes davon zur Herstellung eines Arzneimittels zur Bekämpfung oder Vorbeugung altersbedingter Veränderungen.

7. Pharmazeutisches Präparat, dadurch gekennzeichnet, daß es ein 2-(N-(2-Aminoethyl)amino)-essigsäurederivat der Formel I,

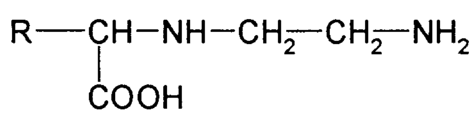

I

worin R Phenyl, Thienyl, Furyl, Pyridyl, Pyrrolyl, Imidazolyl, Thiazolyl oder durch Halogen, Alkyl, Alkoxy, Dialkylaminoalkoxy, Alkoxyalkoxy ein- oder mehrfach substituiertes Phenyl, Thienyl, Furyl, Pyridyl, Pyrrolyl, Imidazolyl oder Thiazolyl bedeutet, oder ein pharmakologisch annehmbares Salz davon oder mehrere derartige Verbindungen als Wirkstoff oder Wirkstoffe zusammen mit einem oder mehreren pharmazeutisch annehmbaren Trägerstoffen und/oder Zusatzstoffen und gegebenenfalls noch ein oder mehrere andere pharmakologische Wirkstoffe enthält.

8. Pharmazeutisches Präparat gemäß Anspruch 7, dadurch gekennzeichnet, daß R Phenyl, Thienyl, Furyl, Pyridyl, Pyrrolyl, Imidazolyl, Thiazolyl oder durch Halogen, ($C_1$-$C_4$)-Alkyl, ($C_1$-$C_4$)-Alkoxy, Di-($C_1$-$C_4$)-alkylamino-($C_1$-$C_4$)-alkoxy, ($C_1$-$C_4$)-Alkoxy-($C_1$-$C_4$)-alkoxy ein- oder mehrfach substituiertes Phenyl, Thienyl, Furyl, Pyridyl, Pyrrolyl, Imidazolyl oder Thiazolyl bedeutet.

9. Pharmazeutisches Präparat gemäß Anspruch 7 und/oder 8, dadurch gekennzeichnet, daß R 2-Thienyl bedeutet.

**Claims**

1. 2-(N-(2-Aminoethyl)amino)acetic acid derivatives of the formula I

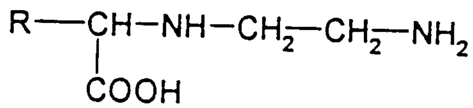

I

in which R denotes phenyl, thienyl, furyl, pyridyl, pyrrolyl, imidazolyl, thiazolyl, or phenyl, thienyl, furyl, pyridyl, pyrrolyl, imidazolyl or thiazolyl which is monosubstituted or polysubstituted by halogen, alkyl, alkoxy, dialkylaminoalkoxy or alkoxyalkoxy, and their pharmacologically acceptable acid addition salts for use as pharmaceuticals.

2. 2-(N-(2-Aminoethyl)amino)acetic acid derivatives of the formula I according to Claim 1, characterised in that R denotes phenyl, thienyl, furyl, pyridyl, pyrrolyl, imidazolyl, thiazolyl, or phenyl, thienyl, furyl, pyridyl, pyrrolyl, imidazolyl or thiazolyl which is monosubstituted or polysubstituted by halogen, ($C_1$-$C_4$)alkyl, ($C_1$-$C_4$)alkoxy, di($C_1$-$C_4$)alkylamino($C_1$-$C_4$)alkoxy or ($C_1$-$C_4$)alkoxy($C_1$-$C_4$)alkoxy, and their pharmacologically acceptable acid addition salts for use as pharmaceuticals.

3. 2-(N-(2-Aminoethyl)amino)acetic acid derivative of the formula I according to Claim 1 and/or 2, characterised in that R denotes 2-thienyl, and its pharmacologically acceptable acid addition salts for use as pharmaceuticals.

4. Use of a 2-(N-(2-aminoethyl)amino)acetic acid derivative of the formula I according to one or more of Claims 1 to 3 or of a pharmacologically acceptable acid addition salt thereof for the production of a pharmaceutical for the control or prevention of damage or diseases which are caused by non-enzymatic glycosylation.

5. Use of a 2-(N-(2-aminoethyl)amino)acetic acid derivative of the formula I according to one or more of Claims 1 to 3 or of a pharmacologically acceptable acid addition salt thereof for the production of a pharmaceutical for the control or prevention of diabetic late damage.

6. Use of a 2-(N-(2-aminoethyl)amino)acetic acid derivative of the formula I according to one or more of Claims 1 to 3 or of a pharmacologically acceptable acid addition salt thereof for the production of a pharmaceutical for the control or prevention of age-related changes.

7. Pharmaceutical preparation, characterised in that it contains a 2-(N-(2-aminoethyl)amino)acetic acid derivative of the formula I

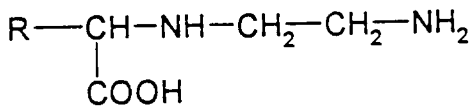

I

in which R denotes phenyl, thienyl, furyl, pyridyl, pyrrolyl, imidazolyl, thiazolyl, or phenyl, thienyl, furyl, pyridyl, pyrrolyl, imidazolyl or thiazolyl which is monosubstituted or polysubstituted by halogen, alkyl, alkoxy, dialkylaminoalkoxy or alkoxyalkoxy, or a pharmacologically acceptable salt thereof or several compounds of this type as an active compound or active compounds, together with one or more pharmaceutically acceptable excipients and/or additives and, if appropriate, additionally one or more other pharmacological active compounds.

8. Pharmaceutical preparation according to Claim 7, characterised in that R denotes phenyl, thienyl, furyl, pyridyl, pyrrolyl, imidazolyl, thiazolyl, or phenyl, thienyl, furyl, pyridyl, pyrrolyl, imidazolyl or thiazolyl which is monosubstituted or poly-substituted by halogen, $(C_1-C_4)$alkyl, $(C_1-C_4)$alkoxy, di$(C_1-C_4)$alkylamino$(C_1-C_4)$alkoxy or $(C_1-C_4)$alkoxy$(C_1-C_4)$alkoxy.

**9.** Pharmaceutical preparation according to Claim 7 and/or 8, characterised in that R denotes 2-thienyl.

**Revendications**

**1.** Dérivés d'acide 2-(N-(2-aminoéthyl)amino)acétique de formule I

$$R-\underset{COOH}{\underset{|}{CH}}-NH-CH_2-CH_2-NH_2 \qquad I$$

dans laquelle R représente le radical phényle, thiényle, furyle, pyridyle, pyrrolyle, imidazolyle ou thiazolyle, ou un radical phényle, thiényle, furyle, pyridyle, pyrrolyle, imidazolyle ou thiazolyle une ou plusieurs fois substitué par un ou des atomes d'halogène ou groupes alkyle, alcoxy, dialkylaminoalcoxy ou alcoxyalcoxy, et leurs sels d'addition avec des acides pharmacologiquement acceptables, pour utilisation en tant que médicaments.

**2.** Dérivés d'acide 2-(N-(2-aminoéthyl)amino)acétique de formule I selon la revendication 1, caractérisés en ce que R représente le radical phényle, thiényle, furyle, pyridyle, pyrrolyle, imidazolyle, thiazolyle ou un radical phényle, thiényle, furyle, pyridyle, pyrrolyle, imidazolyle ou thiazolyle une ou plusieurs fois substitué par un ou des atomes d'halogène ou groupes alkyle en $C_1-C_4$, alcoxy en $C_1-C_4$, dialkyl$(C_1-C_4)$amino-alcoxy$(C_1-C_4)$, alcoxy$(C_1-C_4)$-alcoxy$(C_1-C_4)$, et leurs sels d'addition avec des acides pharmacologiquement acceptables, pour utilisation en tant que médicaments.

**3.** Dérivé d'acide 2-(N-(2-aminoéthyl)amino)acétique de formule I selon la revendication 1 et/ou la revendication 2, caractérisé en ce que R représente le groupe 2-thiényle, et ses sels d'addition avec des acides pharmacologiquement acceptables, pour utilisation en tant que médicament.

**4.** Utilisation d'un dérivé d'acide 2-(N-(2-aminoéthyl)amino)acétique de formule I selon une ou plusieurs des revendications 1 à 3, ou d'un sel d'addition avec un acide pharmacologiquement acceptable de celui-ci, pour la fabrication d'un médicament destiné à combattre ou prévenir des lésions ou maladies qui sont provoquées par la glycosylation non enzymatique.

**5.** Utilisation d'un dérivé d'acide 2-(N-(2-aminoéthyl)amino)acétique de formule I selon une ou plusieurs des revendications 1 à 3, ou d'un sel d'addition avec un acide pharmacologiquement acceptable de celui-ci, pour la fabrication d'un médicament destiné à combattre ou prévenir des lésions diabétiques tardives.

**6.** Utilisation d'un dérivé d'acide 2-(N-(2-aminoéthyl)amino)acétique de formule I selon une ou plusieurs des revendications 1 à 3, ou d'un sel d'addition avec un acide pharmacologiquement acceptable de celui-ci, pour la fabrication d'un médicament destiné à combattre ou prévenir des altérations dues à l'âge.

**7.** Composition pharmaceutique, caractérisée en ce qu'elle contient un dérivé d'acide 2-(N-(2-aminoéthyl)amino)acétique de formule I

$$R-\underset{COOH}{\underset{|}{CH}}-NH-CH_2-CH_2-NH_2 \qquad I$$

dans laquelle R représente le radical phényle, thiényle, furyle, pyridyle, pyrrolyle, imidazolyle ou thiazolyle, ou un radical phényle, thiényle, furyle, pyridyle, pyrrolyle, imidazolyle ou thiazolyle une ou plusieurs fois substitué par un ou des atomes d'halogène ou groupes alkyle, alcoxy, dialkylaminoalcoxy ou alcoxyalcoxy, ou un sel pharmacologiquement acceptable de celui-ci, ou plusieurs composés de ce type, en tant que substance active ou substances actives, conjointement un ou plusieurs véhicules et/ou additifs pharmaceutiquement acceptables et éventuellement encore une ou plusieurs autres substances actives pharmacologiques.

**8.** Composition pharmaceutique selon la revendication 7, caractérisée en ce que R représente un radical phényle,

thiényle, furyle, pyridyle, pyrrolyle, imidazolyle ou thiazolyle, ou un radical phényle, thiényle, furyle, pyridyle, pyrrolyle, imidazolyle ou thiazolyle une ou plusieurs fois substitué par un ou des atomes d'halogène ou groupes alkyle en $C_1$-$C_4$, alcoxy en $C_1$-$C_4$, dialkyl($C_1$-$C_4$)amino-alcoxy($C_1$-$C_4$), alcoxy($C_1$-$C_4$)-alcoxy($C_1$-$C_4$).

**9.** Composition pharmaceutique selon la revendication 7 et/ou la revendication 8, caractérisée en ce que R représente le groupe 2-thiényle.